# EUROPÄISCHE PATENTSCHRIFT

(11) **EP 0 434 057 B1**
(45) Veröffentlichungstag und Bekanntmachung des Hinweises auf die Patenterteilung: **31.07.1996**
(21) Anmeldenummer: 90124954.0
(22) Anmeldetag: 20.12.1990
(51) Int. Cl.: C07D 487/14, A61K 31/40

(54) **Indolocarbazol-Derivate, Verfahren zu deren Herstellung und deren Verwendung**
Indolocarbazole derivatives, processes for their preparation and their use
Dérivés d'indolocarbazole, procédés pour leur préparation et leur utilisation

(30) Priorität: 21.12.1989 DE 3942296
(43) Veröffentlichungstag der Anmeldung: 26.06.1991
(73) Patentinhaber: GÖDECKE AKTIENGESELLSCHAFT, D-10587 Berlin (DE)
(72) Erfinder: Kleinschroth, Jürgen, Dr., W-7819 Denzlingen (DE); Schächtele, Christoph, Dr., W-7800 Freiburg i. Br. (DE); Hartenstein, Johannes, Dr., W-7801 Stegen-Wittental (DE); Rudolph, Claus, Dr., W-7801 Vörstetten (DE)

(56) Entgegenhaltungen:
- EP-A- 0 328 000
- EP-A- 0 370 236
- THE JOURNAL OF ANTIBIOTICS, vol. 40, no. 12, Dezember 1987, Seiten 1782 - 1784; I. TAKAHASHI et al.: "UCN-01, a selective inhibitor of protein kinase C from Streptomyces"
- INTERNATIONAL JOURNAL OF CANCER, vol. 43, 1989, Seiten 851 - 856; T. MEYER et al.:"A derivative of staurosporine [CGP 412551] shows selectivity for protein kinase C inhibition and in vitro anti-proliferative as ..."

## Beschreibung

Die Erfindung betrifft neue Indolocarbazol-Derivate der allgemeinen Formel I 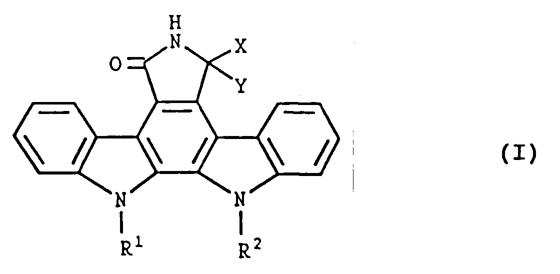 in welcher einer der Reste R¹ und R² für Wasserstoff oder eine geradkettige oder verzweigte Alkylgruppe mit 1 bis 6 C-Atomen steht und der andere der Reste R¹ und R² für eine geradkettige oder verzweigte Cyanoalkyl-, Cyanoalkoxyalkyl-, Cyanoalkylthioalkyl-, Isocyanoalkyl-, oder Azidoalkyl gruppe mit jeweils bis zu 6 C-Atomen steht, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet, sowie deren pharmakologisch unbedenkliche Salze, Verfahren zur Herstellung der Verbindungen I oder von Regioisomerengemischen aus zweien dieser Verbindungen I sowie Arzneimittel mit einem Gehalt an mindestens einer der Verbindungen I.

Bevorzugt werden Verbindungen der allgemeinen Formel I, in welcher einer der Reste R¹ und R² für Wasserstoff, Methyl, Ethyl, Propyl oder Isopropyl steht und der andere der Reste R¹ und R² für Cyanomethyl, 2-Cyanoethyl, 2-Cyanopropyl oder 3-Cyanopropyl steht und X und Y jeweils Wasserstoff bedeuten.

Speziell bevorzugt sind folgende Verbindungen: 12-(2-Cyanoethyl)-6,7,12,13-tetrahydro-13-methyl-5-oxo-5H-indolo[2,3-a]pyrrolo[3,4-c]carbazol (Beispiel 2) und 13-(2-Cyanoethyl)-6,7,12,13-tetrahydro-12-methyl-5-oxo-5H-indolo[2,3-a]pyrrolo[3,4-c]carbazol oder Regioisomerengemische dieser beiden Verbindungen.

Die Herstellung der Verbindungen I erfolgt je nach Substitution nach einem der im folgenden beschriebenen Verfahren:
A) Die Substitution an den Carbazol-N-atomen von Indolocarbazolderivaten der allgemeinen Formel I, in der einer der Reste R¹ und R² für Wasserstoff steht, oder von bekannten Indolocarbazolen der allgemeinen Formel II (zur Herstellung siehe Patentanmeldung EP-A-0 328 000), 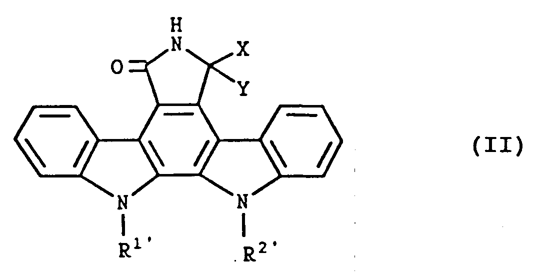 in der X und Y die oben angegebenen Bedeutungen besitzen und R^{1'} und R^{2'} für Wasserstoff stehen, oder einer der Reste R^{1'} und R^{2'} für Wasserstoff steht und der andere der Reste R^{1'} und R^{2'} für eine geradkettige oder verzweigte Alkylgruppe mit 1 bis 6 C-Atomen steht, kann in an sich für die N-Substitution von Indolen oder Carbazolen bekannter Weise durchgeführt werden. Z.B. kann eine geradkettige oder verzweigte Alkylgruppe mit 1 bis 6 C-Atomen in eine Verbindung der allgemeinen Formel I, in der einer der Reste R¹ oder R² für Wasserstoff steht, dadurch eingeführt werden, daß man zuerst diese Verbindung in ein Alkalimetallderivat, z.B. das Natriumderivat überführt, z.B. mittels eines Alkalimetallhydrids, wie Natriumhydrid und dann das erhaltene Derivat mit einer Verbindung der allgemeinen Formel III

   R⁵-Z (III)

   in der R⁵ für eine geradkettige oder verzweigte Alkylgruppe mit 1 bis 6 C-Atomen steht und Z vorzugsweise Halogen, insbesondere Chlor, Brom und Jod bedeutet, umsetzt.
   Verbindungen der allgemeinen Formel I, in der R¹ oder R² für einen Methyl- oder Ethylrest steht, können auch durch Alkylierung mit Dimethyl- oder Diethylsulfat in bekannter Weise hergestellt werden.
   In analoger Weise können Verbindungen der allgemeinen Formel II, in der R^{1'}, R^{2'}, X und Y die oben angegebenen Bedeutungen besitzen, mit Verbindungen der allgemeinen Formel III, in der R⁵ für einen geradkettigen oder verzweigten Cyanoalkyl-, Cyanoalkoxyalkyl-, Cyanoalkylthioalkyl-, Isocyanoalkyl- oder Azidoalkylrest steht und Z die oben angegebenen Bedeutungen besitzt, zu Verbindungen der allgemeinen Formel I umgesetzt werden, in der einer der Reste R¹ oder R² eine der für R⁵ angegebenen Bedeutungen besitzt.
B) Verbindungen der allgemeinen Formel I werden auch erhalten durch basenkatalysierte Michaeladdition von Indolocarbazolen der allgemeinen Formel II, in der X, Y, R^{1'} und R^{2'} die oben angegebenen Bedeutungen besitzen, an aktivierte Olefine der allgemeinen Formel IV 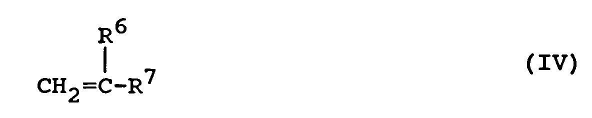 in der R⁶ für Wasserstoff oder eine C1-C3-Alkylgruppe steht und R⁷ eine Cyano gruppe bedeutet.
   Als Basen für die Michaeladditionen nach Verfahren B können Alkalimetallalkoxide, -hydroxide oder -amide, z.B. Natriumethylat, Natriumhydroxid oder Natriumamid; Ammoniak; sekundäre Amine, z.B. Diethylamin, Diisopropylamin oder Piperidin; tertiäre Amine, z.B. Triethylamin, Pyridin oder DBU (1,8-Diazabicyclo[5.4.0]undec-7-en) und quartäre Ammoniumhydroxide oder -alkoxide verwendet werden. Geeignete Lösungsmittel für die Michaeladditionen sind C1-C4-Alkohole, z.B. Methanol, Ethanol, tert.-Butanol; Kohlenwasserstoffe, z.B. Toluol; Ether, z.B. Diethylether oder Dioxan und Acetonitril. Vorzugsweise wird als Base DBU und als Lösungsmittel Acetonitril eingesetzt.
   Bei den Michaeladditionen nach Verfahren B werden vorzugsweise Verbindungen der allgemeinen Formel I erhalten, bei denen R² für den neu eingeführten Rest steht.
D) Verbindungen der allgemeinen Formel I, in der einer der Reste X oder Y für Wasserstoff steht und der andere von den beiden Resten eine Hydroxygruppe bedeutet, können auch in an sich bekannter Weise durch Oxidation von Verbindungen der allgemeinen Formel I, in der X und Y für Wasserstoff stehen, mit Bleitetraacetat in Eisessig hergestellt werden. Wird die Oxidation in Gegenwart von C1-C4-Alkoholen durchgeführt, erhält man Verbindungen der allgemeinen Formel I, in der einer der Reste X oder Y für Wasserstoff steht und der andere von den beiden Resten eine C1-C4-Alkoxygruppe bedeutet.

Sofern nach den beschriebenen Verfahren Regioisomerengemische anfallen, können Verbindungen der allgemeinen Formel I in Form der Regioisomerengemische verwendet werden oder es können die Regioisomeren durch bekannte Trennverfahren wie Kristallisation oder Chromatographie getrennt werden.

Verbindungen der allgemeinen Formel I, die ein chirales Zentrum aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Saure Verbindungen der allgemeinen Formel I können, falls gewünscht, durch Behandlung mit einer geeigneten Base in ein pharmazeutisch verwendbares Salz in an sich bekannter Weise übergeführt werden. Als Basen kommen anorganische Basen, wie z.B. Natrium-, Kalium- oder Calciumsalze oder organische Basen, wie Äthylendiamin oder Mono- oder Diäthanolamin, in Betracht.

Basische Verbindungen der allgemeinen Formel I, welche ein basisches Zentrum an einem der Reste R¹ oder R² aufweisen, werden zum Zwecke der Reinigung und/oder aus galenischen Gründen bevorzugt in kristalline, pharmakologisch verträgliche Salze übergeführt. Die Salze werden in üblicher Weise durch Neutralisation der Basen mit entsprechen den anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage.

Die erfindungsgemäßen Verbindungen sind potente Inhibitoren von Proteinkinasen wie der Proteinkinase C (PKC). So zeigt z.B. die Verbindung von Beispiel 2 im Enzym-Assay der mit Phosphatidylserin und Diacylglycerol aktivierten Proteinkinase C eine 50%ige Inhibierung bei einer Konzentration von 20 nM. Der Versuch wurde gemäß EP-A-0 255 126 (Hemmung von Proteinkinase C) durchgeführt.

Es sind zwar schon Indolocarbazole als Inhibitoren der Proteinkinase C beschrieben worden (J. Antibiot. 1977, 30, 275; Biochem. Biophys. Res. Commun. 1986, 135, 197; Int. J. Cancer 1989, 43, 851; EP-A-0 328 000). Der Vorteil der erfindungsgemäßen Verbindungen der allgemeinen Formel I gegenüber bekannten Indolocarbazolderivaten, die als Inhibitoren der Proteinkinase C beschrieben sind, liegt in der bisher für diese Verbindungsklasse noch nicht beschriebenen hohen Selektivität der PKC-Inhibierung im Vergleich zur Inhibierung anderer Proteinkinasen, wie z.B. der cAMP-abhängigen Proteinkinase (A-Kinase), der cGMP-abhängigen Proteinkinase (G-Kinase), der Myosin Light Chain Kinase (MLC-Kinase) und der tyrosinspezifischen Kinase (T-Kinase).

Die überlegene Selektivität der erfindungsgemäßen Verbindungen geht aus der Tabelle 1 hervor, in der die IC₅₀-Werte für die Verbindung des Beispiels 2 im Vergleich mit den für das Indolocarbazolglykosid Staurosporin beobachteten IC₅₀-Werten aufgelistet sind. Wie der Vergleich zeigt, ist im Gegensatz zu dem unselektiven Inhibitor Staurosporin bei der Verbindung von Beispiel 2 eine Inhibierung der anderen Proteinkinasen erst bei Konzentrationen zu erreichen, die über dem 100fachen der für die Inhibierung der PKC erforderlichen Konzentrationen liegen.

**Tabelle 1**

| Inhibierung von Proteinkinasen (IC₅₀, µM) | | | | | |
|---|---|---|---|---|---|
| | PKC | A- | G- | MLC | T-Kinase |
| Beispiel 2 | 0.020 | >100 | 6.2 | 5.8 | 25 |
| Staurosporin | 0.013 | 0.04 | 0.018 | 0.0096 | 0.006 * |

| | | | | | |
|---|---|---|---|---|---|
| * J. Antibiot. 1987, 40, 1782 | | | | | |

Die Bestimmung der Inhibierung von A-, G-, MLC- oder T-Kinase erfolgte nach den in der Literatur beschriebenen Verfahren (Gill, G.N., Walton M., Advances in cyclic nucleotide research, Vol. 10, herausgegeben von G. Brooker und G.A. Robinson, Raven Press, New York, 1979; Eur. J. Biochem. 1986, 158, 203; Biochem. J. 1984, 218, 863; Analyt. Biochem. 1983, 135, 37; Mol. Immunol. 1989, 26, 897).

Die Proteinkinase C spielt für die intracelluläre Signaltransduktion eine wichtige Schlüsselrolle und ist eng mit der Regulation von kontraktilen, sekretorischen und proliferativen Prozessen verknüpft. Aufgrund dieser Eigenschaften können die erfindungsgemäßen Verbindungen zur Behandlung und/oder Prophylaxe von Herz- und Gefäßkrankheiten wie Thrombosen, Arteriosklerose, Hypertonien, von bronchopulmonären Krankheiten, Entzündungsprozessen, Allergien, Krebs und degenerativen Schäden des Zentralnervensystems und zur Behandlung viraler Erkrankungen verwendet werden. Aufgrund der erhöhten Selektivität der erfindungsgemäßen Verbindungen sind bei der Therapie und/oder Prophylaxe der genannten Krankheiten weniger Nebenwirkungen infolge der Inhibierung anderer Kinasen zu erwarten.

Die Verbindungen können in der jeweils geeigneten Formulierung enteral oder parenteral in Dosen von 1 bis 500 mg/kg, bevorzugt 1 bis 50 mg/kg verabreicht werden.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citrat-Puffer, Ethanol, Komplexbildner (wie Äthylendiamintetraessigsäure und deren nicht-toxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die folgenden Beispiele dienen der näheren Erläuterung der Erfindung:

### Beispiel 1

### 12-(2-Cyanoethyl)-6,7,12,13-tetrahydro-5-oxo-5H-indolo-[2,3-a]pyrrolo[3,4-c]carbazol

300 mg (0,96 mmol) 6,7,12,13-Tetrahydro-5-oxo-5H-indolo-[2,3-a]pyrrolo[3,4-c]carbazol, suspendiert in 20 ml Acetonitril, werden mit 0,3 ml (4,56 mmol) Acrylsäurenitril und 2 Tropfen 1,8-Diazabicyclo[5,4,0]undec-7-en (DBU) versetzt und 3 Tage bei 20°C gerührt. Das Gemisch von Ausgangsmaterial und Produkt wird abfiltriert, mit den gleichen Mengen Acrylsäurenitril, DBU und Acetonitril erneut angesetzt und weitere 3 Tage bei 20°C gerührt. Nach nochmaligem Wiederholen dieses Vorgehens und weiteren 3 Tagen bei 20°C wird 12-(2-Cyanoethyl)-6,7,12,13-tetrahydro-5-oxo-5H-indolo[2,3-a]pyrrolo[3,4-c]carbazol in Form beiger Kristalle abfiltriert, die sich oberhalb 275°C zersetzen.

### Beispiel 2

### 12-(2-Cyanoethyl)-6,7,12,13-tetrahydro-13-methyl-5-oxo-5H-indolo[2,3-a]pyrrolo[3,4-c]carbazol

36 mg (1,2 mmol) Natriumhydrid (80%ig in Mineralöl) werden unter Stickstoffatmosphäre in 50 ml trockenem Dimethylformamid suspendiert und 365 mg (1 mmol) 12-(2-Cyanoethyl)-6,7,12,13-tetrahydro-5-oxo-5H-indolo[2,3-a]pyrrolo[3,4-c]carbazol portionsweise bei Raumtemperatur zugegeben. Nach Abklingen der Gasentwicklung wird 1 Stunde bei 20°C gerührt, dann werden 0,08 ml (1,25 mmol) Methyljodid zugefügt und 16 Stunden bei Raumtemperatur gerührt. Das Lösungsmittel wird im Vakuum abdestilliert und der Rückstand an Kieselgel mit Toluol/Ethanol 95:5 chromatographiert. Die Fraktion mit dem Rf 0,3 in Dichlormethan/Methanol 95:5 wird isoliert und aus Tetrahydrofuran umkristallisiert. Man erhält 12-(2-Cyanoethyl)-6,7,12,13-tetrahydro-13-methyl-5-oxo-5H-indolo[2,3-a]pyrrolo[3,4-c]carbazol in Form blaßgelber Kristalle, die sich oberhalb 270°C zersetzen.

### Beispiel 3

### 12-(2-Cyanoethyl)-6,7,12,13-tetrahydro-7-hydroxy-13-methyl-5-oxo-5H-indolo-[2,3a]pyrrolo[3,4-c]carbazol

140 mg (0,37 mmol) 12-(2-Cyanoethyl)-6,7,12,13-tetrahydro-13-methyl-5-oxo-5H-indolo[2,3-a]pyrrolo[3,4-c]carbazol (Beispiel 2) in 20 ml Eisessig werden mit 197 mg (0,44 nmol) Bleitetraacetat 2 Tage bei 20°C gerührt. Das Lösungsmittel wird im Vakuum abdestilliert und der Rückstand zwischen 50 ml Tetrahydrofuran und 50 ml gesättigter Natriumhydrogencarbonatlösung verteilt. Die Tetrahydrofuranphase wird abgetrennt, mit gesättigter Natriumhydrochloridlösung gewaschen, getrocknet (Natriumsulfat) und eingedampft. Der Rückstand wird an Kieselgel mit Toluol/Ethylacetat 95:5, dann Dichlormethan/Methanol 98/2, als Elutionsmittel chromatographiert. Die Fraktion mit dem Rf 0,1 in Toluol/Ethylacetat 3:1 wird isoliert. Man erhält 12-(2-Cyanoethyl)-6,7,12,13-tetrahydro-7-hydroxy-13-methyl-5-oxo-5H-indolo-[2,3a]pyrrolo[3,4-c]carbazol in Form beiger Kristalle, die sich ab ca. 220°C zersetzen.

### Beispiel 4

### 12-(2-Cyanoethyl)-6,7,12,13-tetrahydro-7-methoxy-13-methyl-5-oxo-5H-indolo-[2,3a]pyrrolo[3,4-c]carbazol

356 mg (0,94 mmol) 12-(2-Cyanoethyl)-6,7,12,13-tetrahydro-13-methyl-5-oxo-5H-indolo[2,3-a]pyrrolo[3,4-c]carbazol (Beispiel 2) in 50 ml Eisessig werden mit 459 mg (1,04 mmol) Bleitetraacetat und 1 ml Methanol 16 Stunden bei 20°C gerührt. Es wird analog Beispiel 4 aufgearbeitet und das Rohprodukt an Kieselgel mit Dichlormethan/Methanol (199:1, v/v) als Elutionsmittel chromatographiert. Die Fraktion mit dem Rf 0,25 in Toluol/Ethylacetat 4:1 wird isoliert. Man erhält 12-(2-Cyanoethyl)-6,7,12,13-tetrahydro-7-methoxy-13-methyl-5-oxo-5H-indolo-[2,3a]pyrrolo[3,4-c]carbazol in Form beiger Kristalle, die sich ab ca. 200°C zersetzen.

## Patentansprüche

1. Indolocarbazol-Derivate der allgemeinen Formel I 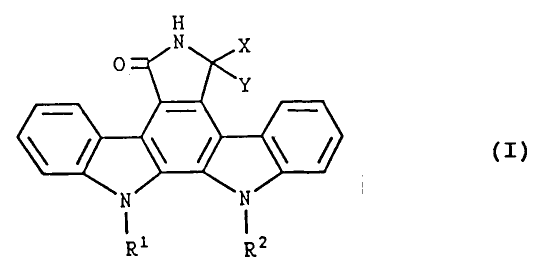 in welcher einer der Reste R¹ und R² für Wasserstoff oder eine geradkettige oder verzweigte Alkylgruppe mit 1 bis 6 C-Atomen steht und der andere der Reste R¹ und R² für eine geradkettige oder verzweigte Cyanoalkyl-, Cyanoalkoxyalkyl-, Cyanoalkylthioalkyl-, Isocyanoalkyl-, oder Azidoalkyl gruppe mit jeweils bis zu 6 C-Atomen steht, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet, sowie deren pharmakologisch unbedenkliche Salze.

2. Indolocarbazol-Derivate der allgemeinen Formel I gemäß Anspruch 1, in welcher einer der Reste R¹ und R² für Wasserstoff, Methyl, Ethyl, Propyl oder Isopropyl steht und der andere der Reste R¹ und R² für Cyanomethyl, 2-Cyanoethyl, 2-Cyanopropyl oder 3-Cyanopropyl steht und X und Y jeweils Wasserstoff bedeuten.

3. Indolocarbazol-Derivate der allgemeinen Formel I gemäß Anspruch 1, nämlich 12-(2-Cyanoethyl)-6,7,12,13-tetrahydro-7-hydroxy-13-methyl-5-oxo-5H-indolo-[2,3a]pyrrolo[3,4-c]carbazol, 12-(2-Cyanoethyl)-6,7,12,13-tetrahydro-7-methoxy-13-methyl-5-oxo-5H-indolo-[2,3a]pyrrolo[3,4-c]carbazol, 12-(2-Cyanoethyl)-6,7,12,13-tetrahydro-13-methyl-5-oxo-5H-indolo[2,3-a]pyrrolo[3,4-c]carbazol und 13-(2-Cyanoethyl)-6,7,12,13-tetrahydro-12-methyl-5-oxo-5H-indolo[2,3-a]pyrrolo[3,4-c]carbazol oder Regioisomerengemische dieser beiden Verbindungen.

4. Verfahren zur Herstellung von Indolocarbazol-Derivaten der allgemeinen Formel I 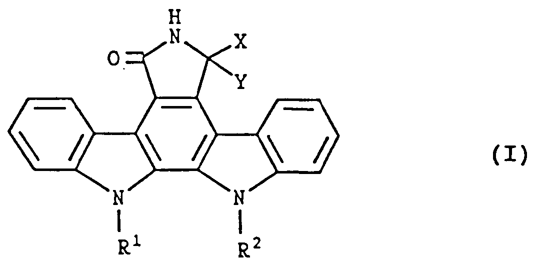 in welcher einer der Reste R¹ und R² für Wasserstoff oder eine geradkettige oder verzweigte Alkylgruppe mit 1 bis 6 C-Atomen steht und der andere der Reste R¹ und R² für eine geradkettige oder verzweigte Cyanoalkyl-, Cyanoalkoxyalkyl-, Cyanoalkylthioalkyl-, Isocyanoalkyl-, Azidoalkyl-gruppe mit jeweils bis zu 6 C-Atomen steht, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet, sowie von deren pharmakologisch unbedenklichen Salzen, dadurch gekennzeichnet, daß je nach Substitution
A) die Substitution an den Carbazol-N-atomen von Indolocarbazolderivaten der allgemeinen Formel I, in der einer der Reste R¹ und R² für Wasserstoff steht, oder von bekannten Indolocarbazolen der allgemeinen Formel II 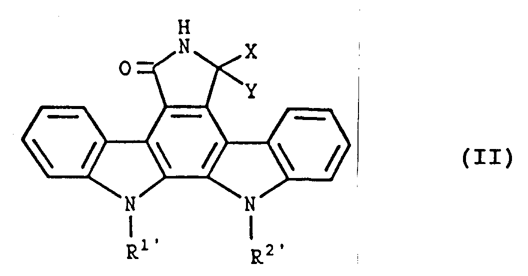 in der X und Y die oben angegebenen Bedeutungen besitzen und R^{1'} und R^{2'} für Wasserstoff stehen, oder einer der Reste R^{1'} und R^{2'} für Wasserstoff steht und der andere der Reste R^{1'} und R^{2'} für eine geradkettige oder verzweigte Alkylgruppe mit 1 bis 6 C-Atomen steht, in an sich für die N-Substitution von Indolen oder Carbazolen bekannter Weise durchgeführt wird, oder
B) die Substitution durch basenkatalysierte Michaeladdition von Indolocarbazolen der allgemeinen Formel II, in der X, Y, R^{1'} und R^{2'} die oben angegebenen Bedeutungen besitzen, an aktivierte Olefine der allgemeinen Formel IV 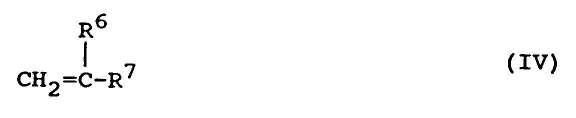 in der R⁶ für Wasserstoff oder eine C1-C3-Alkylgruppe steht und R⁷ eine Cyano gruppe bedeutet, durchgeführt wird oder
D) für Verbindungen der allgemeinen Formel I, in der einer der Reste X und Y für Wasserstoff und der andere für eine Hydroxy- oder C1-C4-Alkoxygruppe steht, durch Oxidation von Verbindungen der allgemeinen Formel I, in denen X und Y für Wasserstoff stehen, mit Bleitetraacetat in Eisessig und für den Fall einer C1-C4-Alkoxygruppe in Gegenwart des entsprechenden C1-C4-Alkohols durchgeführt wird.

5. Verfahren nach Anspruch 4 zur Herstellung von Indolocarbazol-Derivaten der allgemeinen Formel I, in welcher einer der Reste R¹ und R² für Wasserstoff, Methyl, Ethyl, Propyl oder Isopropyl steht und der andere der Reste R¹ und R² für Cyanomethyl, 2-Cyanoethyl, 2-Cyanopropyl, 3-Cyanopropyl, steht und X und Y jeweils Wasserstoff bedeuten.

6. Verfahren nach Anspruch 4 zur Herstellung von Indolocarbazol-Derivaten der allgemeinen Formel I, nämlich:
12-(2-Cyanoethyl)-6,7,12,13-tetrahydro-7-hydroxy-13-methyl-5-oxo-5H-indolo-[2,3a]pyrrolo[3,4-c]carbazol, 12-(2-Cyanoethyl)-6,7,12,13-tetrahydro-7-methoxy-13-methyl-5-oxo-5H-indolo-[2,3a]pyrrolo[3,4-c]carbazol,
12-(2-Cyanoethyl)-6,7,12,13-tetrahydro-13-methyl-5-oxo-5H-indolo[2,3-a]pyrrolo[3,4-c]carbazol und 13-(2-Cyanoethyl)-6,7,12,13-tetrahydro-12-methyl-5-oxo-5H-indolo[2,3-a]pyrrolo[3,4-c]carbazol oder von Regioisomerengemischen dieser beiden Verbindungen.

7. Arzneimittel enthaltend neben üblichen Hilfs- und Zusatzstoffen Verbindungen der allgemeinen Formel I gemäß Anspruch 1 bis 6.

8. Verwendung von Verbindungen der allgemeinen Formel I gemäß Anspruch 1 bis 6 zur Herstellung von Arzneimitteln zur Behandlung und/oder Prophylaxe von Herz- und Gefäßkrankheiten wie Thrombosen, Arteriosklerose, Hypertonien, von bronchopulmonären Krankheiten, Entzündungsprozessen, Allergien, Krebs und degenerativen Schäden des Zentralnervensystems und zur Behandlung viraler Erkrankungen.

## Claims

1. Indolocarbazole derivatives of the general formula I 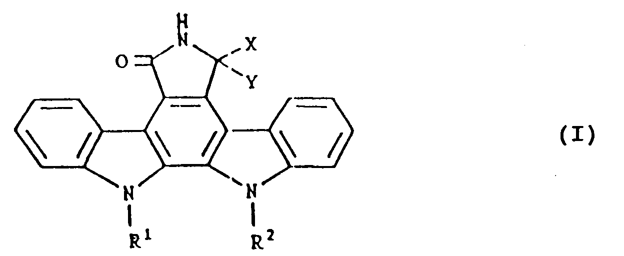 wherein one of the symbols R¹ and R² stands for a hydrogen atom or a straight-chained or branched alkyl radical containing up to 6 carbon atoms and the other Symbol R¹ and R² stands for a straight-chained or branched cyanoalkyl, cyanoalkoxyalkyl, cyanoalkylthioalkyl, isocyanoalkyl, or azidoalkyl radical containing, in each case, up to 6 carbon atoms and X and Y are either the same and both represent hydrogen atoms or X and Y are different, one of the symbols X and Y standing for a hydrogen atom and the other standing for a hydroxyl group or an alkoxy radical containing up to 4 carbon atoms, as well as the pharmacologically acceptable salts thereof.

2. Indolocarbazole derivatives of general formula (I) according to claim 1, wherein one of the symbols R¹ and R² stands for a hydrogen atom or a methyl, ethyl, propyl or isopropyl radical and the other symbol R¹ and R² stands for a cyanomethyl, 2-cyanoethyl, 2-cyanopropyl, or 3-cyanopropyl radical and X and Y are each hydrogen atoms.

3. Indolocarbazole derivatives of general formula (I) according to claim 1, namely,
12-(2-cyanoethyl)-6,7,12,13-tetrahydro-7-hydroxy-13-methyl-5-oxo-5H-indolo[2,3-a]pyrrolo[3,4-c]carbazole;
12-(2-cyanoethyl)-6,7,12,13-tetrahydro-7-methoxy-13-methyl-5-oxo-5H-indolo[2,3-a]pyrrolo[3,4-c]carbazole;
12-(2-cyanoethyl)-6,7,12,13-tetrahydro-13-methyl-5-oxo-5H-indolo[2,3-a]pyrrolo[3,4-c]carbazole;
13-(2-cyanoethyl)-6,7,12,13-tetrahydro-12-methyl-5-oxo-5H-indolo[2,3-a]pyrrolo[3,4-c]carbazole;
and regioisomeric mixtures of these compounds.

4. Process for the preparation of compounds of general formula (I) according to claim 1, 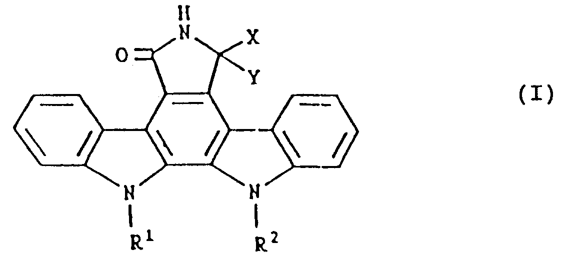 wherein one of the symbols R¹ and R² stands for a hydrogen atom or a straight-chained or branched alkyl radical containing up to 6 carbon atoms and the other Symbol R¹ and R² stands for a straight-chained or branched cyanoalkyl, cyanoalkoxyalkyl, cyanoalkylthioalkyl, isocyanoalkyl, or azidoalkyl radical containing, in each case, up to 6 carbon atoms and X and Y are either the same and both represent hydrogen atoms or X and Y are different, one of the symbols X and Y standing for a hydrogen atom and the other standing for a hydroxyl group or an alkoxy radical containing up to 4 carbon atoms, as well as the pharmacologically acceptable salts thereof.
wherein, depending upon the substitution,
A) the substitution on the carbazole nitrogen atoms of indolocarbazole derivatives of general formula (I), in which one of the symbols R¹ and R² stands for a hydrogen atom, or of known indolocarbazoles of the general formula II 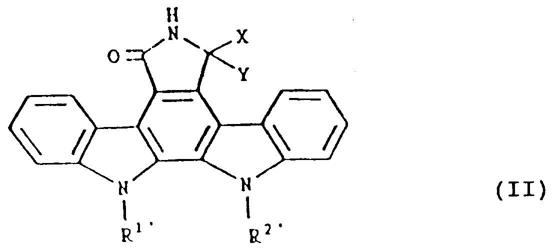 in which X and Y have the above-given meanings and R^{1'} and R^{2'} stands for a hydrogen atom and the other symbol R^{1'} and R^{2'} stands for a straight-chained or branched alkyl radical containing up to 6 carbon atoms, is carried out in a manner known for the N-substitution of indoles or carbazoles; or
B) the substitution is carried out by base-catalysed Michael addition of indolocarbazoles of general formula (II), in which X, Y, R^{1'} and R^{2'} have the above-given meanings, to activated olefins of the general formula (IV) 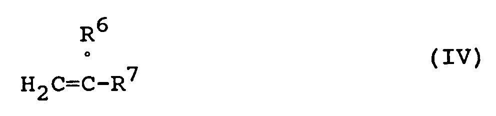 in which R⁶ is a hydrogen atom or a C1-C3-alkyl radical and R⁷ is a cyano group or an unsubstituted or substituted carboxamide group; or
D) for compounds of general formula (I) in which one of the radicals R¹ and R² is hydrogen and the other is a hydroxy or C1-C4 alkoxy radical, by oxidation of compounds of general formula I in which both radicals X and Y are hydrogen, with lead tetraacetate in glacial acetic acid and in case of a C1-C4 alkoxy radical in the presence of the corresponding C1-C4 alcohol.

5. Process according to claim 4 for the preparation of indolocarbazole derivatives of general formula (I), wherein one of the symbols R¹ and R² stands for a hydrogen atom or a methyl, ethyl, propyl or isopropyl radical and the other symbol R¹ and R² stands for a cyanomethyl, 2-cyanoethyl, 2-cyanopropyl, or 3-cyanopropyl radical and X and Y are each hydrogen atoms.

6. Process according to claim 4 for the preparation of indolocarbazole derivatives of general formula (I), namely 12-(2-cyanoethyl)-6,7,12,13-tetrahydro-7-hydroxy-13-methyl-5-oxo-5H-indolo[2,3-a]pyrrolo[3,4-c]carbazole;
12-(2-cyanoethyl)-6,7,12,13-tetrahydro-7-methoxy-13-methyl-5-oxo-5H-indolo[2,3-a]pyrrolo[3,4-c]carbazole;
12-(2-cyanoethyl)-6,7,12,13-tetrahydro-13-methyl-5-oxo-5H-indolo[2,3-a]pyrrolo[3,4-c]carbazole;
13-(2-cyanoethyl)-6,7,12,13-tetrahydro-12-methyl-5-oxo-5H-indolo[2,3-a]pyrrolo[3,4-c]carbazole;
and regioisomeric mixtures of these compounds.

7. Pharmaceutical compositions containing at least one compound of general formula (1) according to claim 1, as well as conventional adjuvant and addition materials.

8. The use of compounds of general formula (I) according to claims 1 to 6 for the preparation of pharmaceutical compositions for the treatment and/or prophylaxis of heart and blood vessel diseases, such as thromboses, arterioscleroses, hypertonias, of bronchopulmonary diseases, inflammatory processes, allergies, cancers and degenerative damage of the central nervous system and for the treatment of viral diseases.

## Revendications

1. Dérivés d'indolocarbazole de formule générale I: 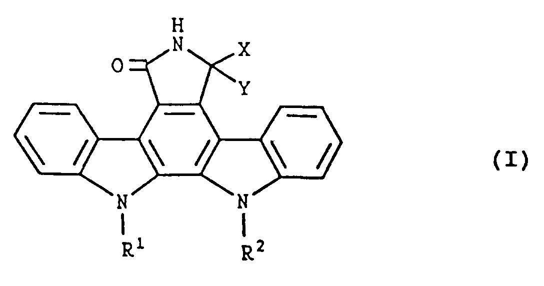 dans laquelle:
un des groupes R¹ et R² représente l'hydrogène ou un groupe alkyle linéaire ou ramifié ayant de 1 à 6 atomes de carbone, et l'autre représente un groupe cyanoalkyle, cyanoalcoxyalkyle, cyanoalkylthioalkyle, isocyanoalkyle ou azidoalkyle, linéaire ou ramifié, avec à chaque fois jusqu'à 6 atomes de carbone;
X et Y soit sont identiques et représentent tout deux l'hydrogène, soit sont différents l'un de l'autre, auquel cas l'un représente l'hydrogène et l'autre représente un groupe hydroxy ou alcoxy ayant de 1 à 4 atomes de carbone;
et leurs sels pharmaceutiquement acceptable

2. Dérivés d'indolocarbazole de formule générale I selon la revendication 1, dans lesquels l'un des groupes R¹ et R² représente l'hydrogène, le méthyle, l'éthyle, le propyle ou l'isopropyle, et l'autre représente un groupe cyanométhyle, 2-cyanoéthyle, 2-cyanopropyle ou 3-cyanopropyle, et les groupes X et Y représentent tous deux l'hydrogène.

3. Dérivés d'indolocarbazole de formule générale I selon la revendication 1, à savoir:
- 12-(2-cyanoéthyl)-6,7,12,13-tétrahydro-7-hydroxy-13-méthyl-5-oxo-5H-indolo-[2,3-a]-pyrrolo-[3,4-c]-carbazole;
- 12-(2-cyanoéthyl)-6,7,12,13-tétrahydro-7-méthoxy-13-méthyl-5-oxo-5H-indolo-[2,3-a]-pyrrolo-[3,4-c]-carbazole;
- 12-(2-cyanoéthyl)-6,7,12,13-tétrahydro-13-méthyl-5-oxo-5H-indolo-[2,3-a]-pyrrolo-[3,4-c]-carbazole; et
- 13-(2-cyanoéthyl)-6,7,12,13-tétrahydro-12-méthyl-5-oxo-5H-indolo-[2,3-a]-pyrrolo-[3,4-c]-carbazole
ou des mélanges de stéréo-isomères de ces composés.

4. Procédé de préparation de dérivés d'indolocarbazole de formule générale I: 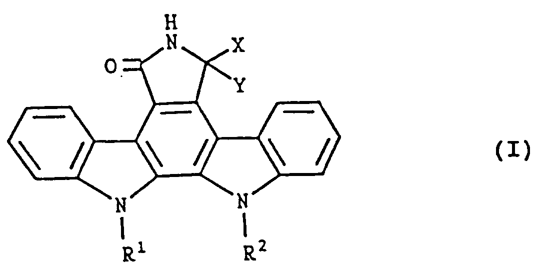 dans laquelle:
un des groupes R¹ et R² représente l'hydrogène ou un groupe alkyle linéaire ou ramifié ayant de 1 à 6 atomes de carbone, et l'autre représente un groupe cyanoalkyle, cyanoalcoxyalkyle, cyanoalkylthioalkyle, isocyanoalkyle ou azidoalkyle, linéaire ou ramifié, avec à chaque fois jusqu'à 6 atomes de carbone;
X et Y soit sont identiques et représentent tout deux l'hydrogène, soit sont différents l'un de l'autre, auquel cas l'un représente l'hydrogène et l'autre représente un groupe hydroxy ou alcoxy ayant de 1 à 4 atomes de carbone;
ainsi que de leurs sels pharmaceutiquement acceptable;
caractérisé en ce qu'il est mis en oeuvre par substitution selon:
A) la substitution sur l'atome d'azote du carbazole de dérivés d'indolocarbazole de formule I, dans laquelle un des groupes R¹ et R² représente l'hydrogène, ou d'indolocarbazoles connus de formule générale II: 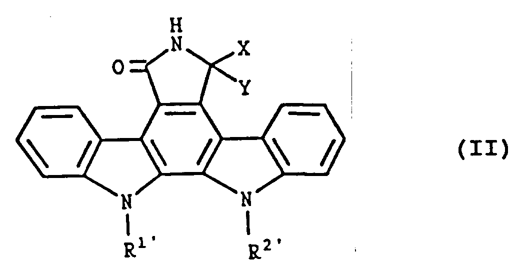 dans laquelle:
X et Y ont la signification ci-dessus, et
R^{1'} et R^{2'} représentent l'hydrogène, ou l'un des groupes R^{1'} et R^{2'} représente l'hydrogène et l'autre représente un groupe alkyle, linéaire ou ramifié, ayant de 1 à 6 atomes de carbone, est mise en oeuvre d'une façon connue pour la substitution sur l'atome d'azote d'indoles ou de carbazoles; ou
B) la substitution est mise en oeuvre par addition de Michaël catalysée par une base, d'indolocarbazoles de formule générale II, dans laquelle X, Y, R^{1'} et R^{2'} ont les significations ci-dessus, sur des oléfines activées de formule IV: 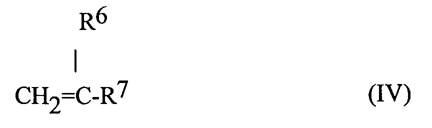 dans laquelle:
R⁶ représente l'hydrogène ou un groupe alkyle en C₁ à C₃, et
R⁷ représente un groupe cyano; ou
D) dans le cas de composés de formule générale I, dans laquelle un des groupes X et Y représente l'hydrogène et l'autre représente un groupe hydroxy ou alcoxy en C₁ à C₄, par oxydation de composés de formule générale I, dans laquelle X et Y représentent l'hydrogène, avec du tétraacétate de plomb dans de l'acide acétique glacial, et dans le cas où l'un des groupes représente un groupe alcoxy en C₁ à C₄ en présence de l'alcool en C₁ à C₄ correspondant.

5. Procédé, selon la revendication 4, de préparation de dérivés d'indolocarbazole de formule générale I, dans laquelle un des groupes R¹ et R² représente l'hydrogène, le méthyle, l'éthyle, le propyle ou l'isopropyle et l'autre représente un groupe cyanométhyle, 2-cyanoéthyle, 3-cyanopropyle, et X et Y représentent tout deux l'hydrogène.

6. Procédé selon la revendication 4, de préparation de dérivés d'indolocarbazole de formule générale I, à savoir:
- 12-(2-cyanoéthyl)-6,7,12,13-tétrahydro-7-hydroxy-13-méthyl-5-oxo-5H-indolo-[2,3-a]-pyrrolo-[3,4-c]-carbazole;
- 12-(2-cyanoéthyl)-6,7,12,13-tétrahydro-7-méthoxy-13-méthyl-5-oxo-5H-indolo-[2,3 a]-pyrrolo-[3,4-c]-carbazole;
- 12-(2-cyanoéthyl)-6,7,12,13-tétrahydro-13-méthyl-5-oxo-5H-indolo-[2,3-a]-pyrrolo-[3,4-c]-carbazole; et
- 13-(2-cyanoéthyl)-6,7,12,13-tétrahydro-12-méthyl-5-oxo-5H-indolo-[2,3-a]-pyrrolo-[3,4-c]-carbazole
ou de mélanges de stéréo-isomères de ces composés.

7. Préparation pharmaceutique contenant, outre des agents et additifs classiques, des composés de formule générale I selon les revendications 1 à 6.

8. Utilisation de composés de formule générale I selon les revendications 1 à 6, pour la préparation de médicaments pour le traitement et/ou la prophylaxie de maladies cardiaques et vasculaires, telles que la thrombose, l'artériosclérose, l'hypertension, de maladies bronchopulmonaires, de maladies inflammatoires, d'allergies, du cancer et des pathologies dégénératives du système nerveux central et pour le traitement de maladies virales.
